# EUROPEAN PATENT APPLICATION

(11) **EP 1 982 727 A1**
(43) Date of publication of application: **22.10.2008**
(21) Application number: 07007759.9
(22) Date of filing: 17.04.2007
(51) Int. Cl.: A61K 39/145, C12N 7/00, C08B 5/14, C08L 1/16

(54) **Method for purification of viral proteins**

(71) Applicant: Max-Planck-Gesellschaft zur Förderung der Wissenschaften e.V., 80539 München (DE)
(72) Inventor: Wolff, Michael, Dr., 39175 Biederitz (DE); Reichl, Udo, Prof. Dr.-Ing., 39104 Magdeburg (DE); Opitz, Lars, Dipl.-Ing., 04928 Döllingen (DE)
(74) Representative: Kröncke, Rolf

(57) **Abstract**

The present invention relates to methods for obtaining viral proteins. In particular, the present invention relates to the purification of viral proteins, in particular, of viral membrane proteins from host cells or genetically engineered cells expressing viral proteins, in particular, viral membrane proteins. Specifically, the present invention provides new methods wherein the host cells expressing viral membrane proteins recombinantly or host cells infected with virus and propagating the same are treated with surfactants for membrane solubilization of said host cells.

## Description

The present invention relates to methods for obtaining viral proteins. In particular, the present invention relates to the purification of viral proteins, in particular, of viral membrane proteins from host cells or genetically engineered cells expressing viral proteins, in particular, viral membrane proteins. Specifically, the present invention provides new approaches wherein the host cells expressing viral membrane proteins recombinantly or host cells infected with virus and propagating the same are treated with surfactants for membrane solubilization of said host cells.

### Technological background

Isolation of appropriate viral protein is most important for the production of vaccines as a prophylactic or therapeutic vaccine against viral infections. Typically, the immune response elicited by virus vaccines is based on raising an appropriate cellular and humoral immunity against immunogens of the virus. Major immunogens of virus are typically membrane proteins or surface proteins. For example, for HIV gp120 and gp41 are described as potential vaccine candidates while for influenza virus, the envelop proteins or surface proteins neuraminidase (NA), hemagglutinin (HA) and the ion channel (M2) are regarded as the most prominent candidate vaccines.

Influenza infection is a wide spread contagious disease of the respiratory tract. Due to annual death rates and its potential to cause pandemics, influenza remains a major public health concern. To control influenza outbreaks prophylactic vaccinations in conjunction with anti-viral medications are regarded as being most promising. Today most commercially available influenza vaccines are derived from influenza viruses produced in the allontoic fluid of embryonated chicken eggs.

Currently there are two main types of inactivated influenza vaccines on the market, namely subunit vaccines and split vaccines. Split vaccines are prepared by the treatment of virus particle with solubilizing concentrations of detergents and subsequent removal of the detergent and of the bulk of the viral lipid material.

Subunit vaccines against influenza, contain primarily the surface protein hemagglutinin, which is responsible for eliciting the desired immune response upon vaccination.

Beside the subunit and split vaccines there are also active and inactivated whole virus vaccines available.

As indicated above, the influenza viruses for vaccines are currently derived mainly from embryonated chicken eggs. However said type of influenza vaccine production is laborious and requires a large quantity of eggs to obtain suitable amounts of vaccines. For example in the presently favoured method of producing influenza vaccines, approximately one egg per trivalent dose is required. Thus, an enormous amount of eggs are necessary and there may arise a supply shortage especially in case of pandemic outbreaks. Even the demand for seasonal vaccines can exceed the vaccine supply as e.g. in 2005/2006. Existing production capacities and processes are normally designed to provide annual influenza epidemics vaccines, however, peaks in the demands for influenza vaccines cannot be complied with by the methods known in the art. Further, allergic reactions may be provoked when administering vaccines derived from chicken eggs. In addition, the quality of the obtained vaccines from the eggs has the potential to vary significantly.

Consequently, there is still a demand for improvements in the production of viral proteins suitable as vaccines. Improvements could not only have a beneficial impact on the short term delivery of vaccines during a pandemic, but could potentially also help to overcome the shortage in influenza vaccines on the global market.

One possibility is the cultivation and fermentation of viral infected host cells or genetically engineered cells expressing recombinant protein. In EP 0 870 508 methods are described allowing isolation and production of surface antigen proteins from influenza viruses propagated in animal cell culture. The method comprises the step of treating the whole virus containing fluid obtained from the cell culture with a DNA digesting enzyme and adding a cationic detergent followed by isolation of the surface antigen proteins by conventional methods based on different types of chromatography. Also other methods described for the purification of viral membrane proteins are based on using culture supernatants from fermentation or virus infected host cells. In US 4,064,232 a process for isolating the immunogenic components of influenza viruses is described. The method is based on treating influenza virions in an aqueous medium with a cationic detergent to selectively solubilise components, and separating the resulting solublilised components from residual sub-viral particles.

However, a main problem also with the cell culture derived virus particles are the low yield of virus membrane proteins. Thus, there is still a need for new methods allowing production of isolated viral membrane proteins e.g. for the production of vaccines with high yields while being cost effective and less laborious. The present invention presents a modified downstream process of cell culture virus particles allowing a significant increase in the production of isolated viral membrane proteins.

### Summary of the present invention

The problem is solved by methods as defined in the claims. That is, the above problems are solved by providing new methods for obtaining isolated viral proteins, in particular, glycoproteins comprising the step of
i) providing host cells containing viral proteins optionally with culture media;
ii) adding at least one surfactant to the host cells of i) and incubating the same for membrane solubilization; and
iii) purification of the viral proteins.

In a preferred embodiment, the method according to the present invention relates to the isolation of viral membrane proteins.

In a preferred embodiment the method further comprises the step of demicellation of the solubilized membranes containing molecules, like membrane molecules, derived from the host cells and the virus.

The purification step of the method according to the present invention comprises preferably the step of hydrophobic interaction chromatography.

In addition, in another preferred embodiment, the method comprises a purification step of affinity chromatography using cellulose having a sulfate ester as a functional group. For example, the cellufine^{™} sulfate is particularly suitable for this affinity chromatography purification step.

In a further preferred embodiment, the above purification step with modified cellulose having sulfate ester functionality is combined with a lectin-affinity chromatography as an example of a ligand specific affinity chromatography.

The present invention further relates to the use of the virus membrane proteins obtained with the methods according to the present invention for the preparation of pharmaceuticals, in particular, of vaccines.

The methods according to the present invention are useful when said methods are performed in disposable or single-use bioreactors and in stainless steel bioreactors since the surfactant can be added directly into the bioreactors allowing solubilization of the membranes of the host cells infected with the virus as well as of virus particles attached to the host cells and present in the supernatant.

Furthermore, the present invention relates to a method for preparing sulphated reinforced cellulose membranes useful in affinity purification of viral proteins.

### Brief description of the drawings

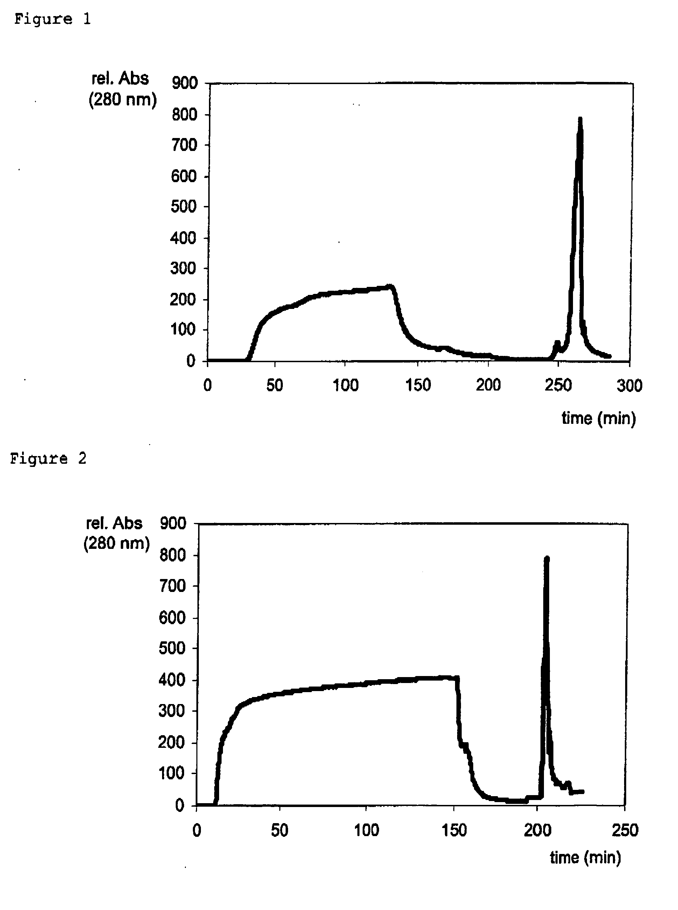

Figure 1: Chromatogram of MDCK-cell derived human influenza virus A/Puerto Rico/8/34, H1N1 hemagglutinin. Total protein was monitored by UV-adsorption at 280 nm. The sample was loaded in PBS onto a 24 ml cellufine^{™} sulphate (Chisso Corporation) column at room temperature with a flow rate of 0.5 ml/min. Bound proteins including hemagglutinin were eluted with 1.2 M NaCl.
Figure 2: Chromatogram of the eluted hemagglutinin fractions after a cellufine^{™} sulphate chromatography. Total protein was monitored by UV-adsorption at 280 nm. The sample was loaded after dialysis against PBS onto a 3 ml EEI.-Adsorbent (Galab Technologies GmbH) column at 4°C with a flow rate of 0.2 ml/min.

Hemagglutinin was eluted in two steps with 0.5 M lactose in PBS and 0.5 M lactose containing 2 M NaCl in PBS.

### Detailed description of the present invention

The present invention relates to methods for obtaining isolated viral proteins from cultures containing host cells, comprising the steps of:
a) providing host cells containing viral proteins optionally with culture media;
b) adding at least one surfactant to the host cells of a) and incubating the same for membrane solubilization; and
c) purification of the viral proteins.

As used herein, the term "host cells" comprises on the one hand genetically engineered cells expressing virus proteins recombinantly, and, on the other hand, cells productively infected with virus.

Preferably, the viral proteins are viral membrane proteins or viral surface proteins.

An amphiphile is defined to be a compound that contains both hydrophilic and a lipophilic group. A surfactant is an amphiphile that possesses two additional properties. First, it significantly modifies the interfacial physics of an aqueous phase (and not only the air-water but also the oil-water and solid-water interfaces) at unusual low concentrations compared to non-surfactants. Second, surfactant molecules associate reversibly with each other (and with numerous other molecules) to a highly exaggregated degree to form thermodynamically stable, macroscopical one-phase, solutions of aggregates or micelles.

The term "surfactant" is derived from "surface active agent". They are soluble both organic solvents and water. In the following, the term "surfactant" is used in the general meaning of surface active agent. Surfactants are also typically classified into four primary groups, namely, anionic, cationic, non-ionic and zwitterionic surfactants.

Viruses are packets of infectious nucleic acid surrounded by protective coats. Typically, the viral nucleic acid is covered by a protein capsid, which protects it from enzymatic attack and mechanical breakage and delivers it to a susceptible host. In some of the more complex viruses, the capsid itself is surrounded by an envelope containing membrane lipids and glycoproteins. For example, the body of the influenza virus has a size of about 75-120 nm and it consists of a core of ribonucleic acid (RNA) associated with the nucleoprotein, surrounded by a viral envelope with a lipid bilayer structure. Typically, the inner layer of the viral envelope is composed to be dominantly of matrix proteins and the outer layer contains most of the host-derived lipid material, like membrane proteins. The influenza virus envelope contains predominantly the membrane proteins neuramidase (NA) and hemagglutinin (HA) beside other proteins like e.g. the ion channel forming glycoprotein M2. HA and NA are transmembrane glycoproteins, whereas the matrix protein lies on the inner surface of the membrane envelope. The ideal influenza vaccine should, contain at least the two essential immunogens, hemagglutinin and neuramidase, in the absence or substantial absence of non-essential components of the viral particle. Thus, in a preferred embodiment the method according to the present invention is directed to obtain the immunogens of the influenza virus, namely hemagglutinin and/or neuramidase and/or M2.

Thus, preferably, the viral proteins are viral membrane proteins.

In case of HIV, another member of the genus of lentivirus, gp120 and gp41 are relevant molecules for vaccine purposes.

Contrary to the prior art techniques, the method according to the present invention does not require to isolate viral particles or to isolate the supernatants from the culture. It is not necessary to isolate the viral particle from cellular debris of the host cells, the host cells themselves and, in the case of adherent cells, microcarriers present in the culture medium. Therefore, one of the major advantages of the present method is the simplification of the process and, thus, avoiding laborious isolation steps to obtain viral membrane proteins.

The method of the present invention further relates to disposable bioreactors and stainless steel stirred tanks. By simply adding at least one surfactant to the crude culture media containing both supernatant and host cells, thus, solubilizing the membranes of the virus particles and the host cells.

After solubilization and, optionally, demicellation and/or protease treatment, the viral membrane proteins may be separated from the remaining cell debris, microcarriers etc. by suitable filtration methods, e.g. ultrafiltration.

The method according to the present invention is versatile insofar that not only adherent cells but also suspension cells or genetically modified transformed cells may be used as host cells using the same process.

In a preferred embodiment of the present invention the method further comprises the step of ultrafiltration of the solution containing the solubilized proteins.

Ultafiltration may be conducted by known methods including cross flow filtration and diafiltration with molecular weight cut offs ranging from 5000 to 50,000 Da.

If necessary, centrifugation steps may be conducted for separation purposes.

In a particular embodiment, the method according to the present invention further comprises the step of protease treatment. Said protease treatment, e.g. treatment with trypsin and bromelain may be conducted directly after culture in the bioreactor, after membrane solubilization, after the ultrafiltration step and/or after a hydrophobic interaction chromatography step following the ultrafiltration step. Preferably, the protease treatment is conducted before ultrafiltration of the cell culture broth.

In another embodiment, the step of demicellation is conducted after membrane solubilization. In this step the solution is treated with an agent, e.g. cetyltrimethylammonium bromide or other demicelling agents known in the art to reduce or decrease formation of micelles of surfactants in the solution.

After ultrafiltration the solution containing the viral membrane proteins is treated by hydrophobic interaction chromatography. This step enables to reduce and/or eliminate the surfactant. For example Amberlite XAD-4 is a preferred hydrophobic compound to be used according to the present invention. Preferably, diafiltration or dialysis is performed in advance of the hydrophobic interaction chromatography.

The diafiltration and/or ultrafiltration is performed e.g. with a membrane having a molecular weight cut-off of 50 000 Da.

The method according to the present invention may further comprise the step of affinity separation, like affinity purification by affinity column chromatography or affinity membrane chromatography using a modified cellulose having sulfate groups, e.g. sulphate ester groups or with sulphuric acid ester crosslinked polysaccharide after the hydrophobic interaction chromatography step. Matrices of modified cellulose having sulfate (ester) groups are described in the art and are commercially available, e.g. cellufine^{™} sulfate. The sulphuric acid ester of cellulose to be used in the present invention includes a sulphuric acid ester of crystalline cellulose or cellulose having crystalline area and non-crystalline area. These starting celluloses are commercially available for example as cellufine^{™} sulfate.

The sulphuric acid ester of a crosslinked polysaccharide to be used in the present invention includes a sulphuric acid ester of polysaccharide, such as dextran, cellulose, argarose, which is crosslinked with a crosslinking agent. The crosslinked polysaccharides are commercially available, for example, as crosslinked dextran such as Sephadex, Sepharose or cellufine.

Alternatively, a modified reinforced cellulose membrane for an affinity separation by membrane chromatography can be obtained among other procedures as follows: To pyridine (20 ml) is added dropwise chlorosulfonic acid (1.12 ml) below 0 °C. After the addition, the mixture is heated to 65 °C while stirring and than cooled to 35 °C. To the mixture are added 30 regenerated cellulose membranes (e.g. RC 55 membrane filters, Whatman or RC - fleece reinforced, Sartorius) and the mixture is stirred at 35 °C for 6 hours. After the reaction, the mixture is cooled to room temperature and then neutralized with aqueous sodium hydroxide. Subsequently, the membrane is washed well with phosphate buffered saline solution, thus, obtaining functionalized cellulose having functional sulphate ester groups.

Thus, the present invention relates also to a method for the preparation of sulphated reinforced cellulose membrane as described above.

The method according to the present invention for preparing the sulphated, preferably reinforced, cellulose membrane comprises the steps of adding chlorosulfonic acid to pyridine at a temperature below 0°C; reacting said mixture at a temperature above 60°C; cooling the mixture at a temperature below 40°C; adding cellulose membrane, in particular regenerated cellulose membrane, to said mixture and reacting the same at a temperature below 40°C, thus, obtaining a sulphated cellulose membrane.

Preferably, the reaction between chlorosulfonic acid and pyridine is conducted at a temperature above 65°C, e.g. at a range of from 60°C to 80°C, like 6°C to 70°C.

The mixture of pyridine and chlorosulfonic acid is cooled at a temperature below 40°C, e.g. at a temperature in the range of from 40°C to 10°C, like 35°C to 20°C, in particular, 35°C to 30°C. Further, the temperature for reacting the mixture of pyridine and chlorosulfonic acid with the cellulose membrane, in particular, the regenerated cellulose membrane, like a reinforced cellulose membrane is below 40°C, e.g. at a temperature in the range of from 40°C to 10°C, like 35°C to 20°C, in particular, 35°C to 30°C.

As an alternative for the above mentioned cellulose having sulfate or sulphate ester groups or additionally, membrane filters having immobilized heparin, heparan sulfate or sulfate dextran may be used.

Further preferred, the method according to the present invention comprises the step of purification of the viral membrane proteins with lectin-affinity chromatography. For example, lectin-affinity chromatography is described in Opitz et al., Vaccine, 2007, 25(5), 939-47 which is incorporated herein by reference. Preferably, the lectin is *Erythrina cristagalli* lectin (ECL) or *Euonymus europaeus* lectin (EEL).

Thus, in a particular preferred embodiment, the method for obtaining isolated viral proteins from cultures containing host cells comprise the steps of:
a) providing host cells containing viral proteins optionally with culture media;
b) adding at least one surfactant to the host cells of a) and incubating the same for membrane solubilization; and
c) purification of the viral proteins comprising the steps of i) optionally hydrophobic interaction chromatography, ii) affinity chromatography using a modified cellulose having sulfate ester groups, and iii) lectin-affinity chromatography or other specific ligands as e.g. sialic acids or sialic acid derivatives like neuraminidase inhibitors.

The method is particular useful for the isolation of viral membrane proteins

The order of the affinity chromatography using modified cellulose having sulfate ester groups and the more specific affinity chromatography, e.g. the lectin chromatography, is not relevant.

Depending on the elution methods of the individual chromatography steps, arises a need for an ultrafiltration, diafiltration or dialysis of the product fraction to remove the product releasing agents as e.g. NaCl.

In addition, the method according to the present invention may comprise further purification steps, like applying a heparin-affinity chromatography, and/or an ionexchange chromatography after the hydrophobic interaction chromatography.

Further, the method may comprise a suffodextran-affinity chromatography after hydrophobic interaction chromatography which may substitute the affinity chromatography using a modified cellulose having sulfate ester groups as indicated above.

Moreover, the purification step may comprise an affinity chromatography using specific (monoclonal) antibody directed to the viral membrane proteins to be purified in addition to or instead of the affinity chromatography using a modified cellulose having sulfate ester groups.

Likewise, the purification step may comprise an affinity chromatography using specific ligands e.g. sialic acids or silaic acid derivatives directed to the viral membrane proteins to be purified in addition to or instead of the affinity chromatography using a modified cellulose having sulfate ester groups, lectins or specific antibodies.

The above mentioned affinity chromatography steps are known in the art and the skilled person is able to select the appropriate type of chromatography and the conditions therefore.

The method according to the present invention is particularly useful for obtaining viral membrane proteins from influenza virus useful as influenza vaccines. That is, the method of the invention may suitable be applied to influenza type A, B or C viruses or mixtures thereof. The particular strain employed will of course depend on the immunity desired from the immunogens to be isolated. Preferably, the influenza virus is a virus responsible for human influenza, swine influenza, avian flu, equine influenza, etc.

Further preferred enveloped viruses are retroviruses (e.g. human immunodeficiency virus), rhabdoviruses, other orthomyxoviruses than influenza, paramyxoviruses, filoviruses (e.g. Ebola), alphaviruses, herpesviruses, and poxviruses (e.g. vaccinia).

Host cell suitable for propagating the virus are well known in the art. For example, suitable host cells for infection and production of viral particles are Madin Darby canine kidney (MDCK) cells (e.g. ECACC deposit number 841211903) cells as described for example in Opitz et al, supra, Genzel et al., Vaccine, 2006, 24, 3261-3272, which are incorporated herein by reference., Vero cells (obtainable from ATCC) and Per.C6 cells (as described in WO01/38362 and WO02/40665, and as deposited under ECACC deposit number 96022940).

Alternatively, the host cells are genetically modified expressing the viral protein, e.g the viral membrane protein. Various expression systems are known in the art. For example, expression of viral membrane proteins in the baculovirus expression system is described in US 5,858,368.

Particularly preferred, the present invention relates to production of influenza virus hemagglutinin, neuramidase, and M2. The influenza virus is particular preferred a human influenza virus causing flu or influenza in human, swine, bird, horse etc.

One major aspect of the present invention is adding at least one surfactant to the culture broth containing host cells propagating the virus or expressing recombinant virus proteins and, optionally, supernatant containing virus particles. Typically virus particles in the supernatant of the culture broth contain membrane molecules derived from the host cell.

Thus, the addition of the at least one surfactant solubilizes the viral membrane proteins present in the cell membrane of the host cells, the free virus particles and the virus particles bound to the host cells. Useful surfactants include anionic, cationic, non-ionic and zwitter-ionic surfactants. The classification of the surfactant is done by the presence of formally charged groups in its head. A non-ionic surfactant has no charge groups in its head. The head of an ionic surfactant carries a net charge. If the charge is negative, the surfactant is more specifically called anionic; if the charge is positive, it is called cationic. If a surfactant contains a head with two oppositely charged groups, it is termed "zwitterionic".

Some commonly encountered surfactants of each type include:

ionic surfactants: i.) anionic surfactants (typically based on sulfate, sulfonate or carboxynate anions) e.g. α-olefin sulphate, ammonium octyl/decyl ether sulphate, sodium sulfosuccinates, sodium tridecyl ether sulphate, triethanolamine lauryl sulphate, sodium glyocholate, sodium taurocholate, sodium taurodeoxycholate, N-lauroylsarcosine, alkyl sulfate salts, especially alkali metal or earth alkali metal alkyl sulfate salts, like sodium dodecyl sulfate, lithium dodecyl sulfate etc. or ammonium lauryl sulfate; sodium laureth sulfate, alkyl benzene sulfonate, deoxycholic acid alkali or earth alkali salts or deoxycholic acids, phosphoric acid esters- and salts , ii.) cationic surfactants e.g. based on quaternary ammonium cations like cetyltrimethylammonium bromide or other alkyl trimethylammonium salts, alkyl amine salts like stearyl amine acetate or coconut alkyl amine acetate, benzalkonium chlorides and bromides, for example benzethonium chloride or methylbenzethonium chloride, stearylaminepolyglycolether or oleylaminepolyglycolether.

Zwitterionic (amphoteric) surfactants, like dodecylbetain, dodecyldimethylaminoxid, CHAPS, CHAPSO, BigCHAP, EMPIGEN BB (N-Dodecyl-N,N-dimethylglycine), Lauryldimethylamineoxid, zwittergent 3-08, zwittergent 3-10, zwittergent 3-12, zwittergent 3-14, zwittergent 3-16, etc. or non-ionic surfactants, like alkylpoly ethylene oxid, alkyl polyglycoside, including: octyl glycoside and decyl maltoside, e.g. nonidet P10 or nonidet P40 surfactants, MEGA-8, -9 or -10, Triton X 100 and related surfactants or surfactants of the Tween family, like Tween 20, Tween 40, Tween 60, Tween 80, APO-10, APO-12, C₈E₆, C₁₀E₆, C₁₂E₆, C₁₂E₈, C₁₂E₉, C₁₂E₁₀, C₁₆E₁₂, C₁₆E₂₁, Heptane-1,2,3-triol, lubrol PX, genapol family, n,-Dodecyl-b-D-glucopyranoside, thesit, pluronic family, etc.

Particulary preferred, the surfactant is a combination of at least two surfactants. Preferably one surfactant is a cationic surfactant while the at least one further surfactant is a non-ionic surfactant. Particularly preferred the surfactant is a combination of cetyltrimethylammonium bromide as cationic surfactant and polysorbate, e.g. Tween 20 or Tween 80, as non-ionic surfactant.

The surfactant enables achieving sufficient solubilization of the viral protein,e.g. the viral membrane protein from the membranes of the host cell or the free virus particle or the host cell bound virus particles.

As mentioned before, the solubilization with the surfactant may be accompanied by protease treatment to further support the release of the viral proteins, e.g. the viral membrane proteins from the surrounding membrane components.

Further, the purification step using lectin-affinity chromatography allows to isolate specifically viral membrane proteins. In addition, by selecting specific ligands, namely specific lectins, it is possible to differentiate between glycoproteins with fully elaborated glycan structures and glycoproteins with different terminal oligosaccharides or not having any carbohydrate moiety.

As mentioned before, the viral membrane proteins obtained according to the present invention are particularly useful as vaccines against virus diseases, like influenza viruses. The skilled person is well aware of formulating the obtained purified viral membrane protein into pharmaceutical composition useful as vaccines. The administration of the pharmaceutical composition, like vaccine, can be done in a variety of ways including, but not limited to, orally, subcutaneously, transcutaneously, intradermally, intravenously, intra-arterial, intra-nodal, intramedullary, intrathecal, intraventricular, intranasaly, intrabronchial, intrarectally, intraperitonally, intramusculary, intrapulmonary, vaginally, rectally, or intraoculary.

Thus, the present invention relates further to a vaccine containing influenza surface antigens from influenza viruses whereby said influenza surface antigen is obtained by any one the methods according to the present invention.

The following examples illustrate the invention further without limiting the same to the specific embodiments described in the examples.

### Examples

### Example 1

Purification of the viral membrane protein hemagglutinin from influenza.

MDCK cells from ECACC, UK, Nr. 841211903 are initially grown in roller bottles (Greiner, Germany, 850 cm²) containing 250 ml GMEM basal medium (Invitrogen/Gibco, #22100-093). After the cells reach confluence they are washed with PBS, trypsinized and applied as an inocculum ((2-3) x10⁵ cells/ml) for a 5l bioreactor (B. Braun Biotech) containing the same media and 2.0 g/l Cytodex 1 solid microcarriers (Amersham Biosciences). The cells are cultivated under standard conditions for 4 days, washed with phosphate buffered saline (PBS) and infected with Influenza A PuertoRico/8/34, H1N1 (NIBSC #99-716) at a MOI of 0.025. The infected cultures are maintained for 48 to 72 h in virus maintenance medium under standard conditions as described in Genzel et al., Vaccine, 2004, 22, 2202-2208. After cultivation, cells and microcarriers as well as cell agglomerates are separated from the medium by sedimentation. The sediment is than subjected to chemical inactivation with 12 mM β-propiolactone (Serva Electrophoresis) for 24 h at 37°C.

Then, polysorbate 80 (0,025 %, w/v final conc.) and cethyltrimethylammonium bromide (0.05 %, w/v final conc.) was added to the sediment and the mixture is incubated overnight at 4 °C while agitating to solubilize the viral membrane protein from the lipid membranes of the cellular and viral components.

Next, equimolar amounts of sodium cholate compared to cethyltrimethylammonium bromide are added to the mixture, thus, reducing formation of micelles of the surfactants. The mixture is incubated for another 12 h at 4 °C while agitating.

Thereafter, the mixture is centrifugated for 10 min with 9000g. Subsequently, the supernatant is diafiltrated against a Euonymus europaeus lectin (EEL) column loading buffer (150mM NaCl, 50 mM Tris, 0.1 mM MnCl₂ and 0.1mM CaCl₂, pH 7.4) using a membrane with a MWCO of 30,000 Da (Millipore) to concentrate the mixture and to remove salts and free surfactants.

Afterwards, the obtained solution is incubated with Amberlite XAD-4 to reduce the amount of surfactant. After performing a hydrophobic interaction chromatography with Amberlite XAD-4, the protein solution is centrifugated for 10 min at 9000g. As the next step, lectin-affinity chromatography is performed. In particular, the sample is loaded to a 3 ml EEL-polymer (Galab Technology GmbH, Germany) column with a flow rate of 0.2 ml/min at 4°C or at room temperature. The target protein is eluted after a column wash in two steps with 0.5 M lactose in column loading buffer and 0.5 M lactose in addition to 1.850 M NaCl in column loading buffer with a flow rate of 0.5 ml/min at 4°C or at room temperature.

The eluted fractions are pooled and diafiltrated against PBS using a membrane with a MWCO of 30,000 Da (Millipore) to remove lactose and salts. Alternatively, diafltration can be performed in a Vivaspin 1R Hydrosart with a MWCO of 30.000 Da (Sartorius).

### Example 2

As example 1, but with modified detergent concentrations
a. Final detergent concentrations: Polysorbate 80: 0.5%
   Cetyltrimethylammonium Bromid: 1.0%
b. Final detergent concentrations: Polysorbate 80: 0.0375%
   Cetyltrimethylammonium Bromid: 0.075%
c. Final detergent concentrations: Polysorbate 80: 0.05%
   Cetyltrimethylammonium Bromid: 0.1%
d. Final detergent concentrations: Polysorbate 80: 0.1 %
   Cetyltrimethylammonium Bromid: 0.2%

### Example 3

The steps conducted in example 1 are repeated except that a modified purification matrix is used
Namely, instead of the EEL-polymer alternative chromatography media based on porous glass (Trisopor, VitraBio GmbH), on agarose (Agarose, Vector Laboratories Inc.; Actigel ALD, Sterogene Bioseparations Inc., USA), or on cellulose (Cellufine, Chisso Cor., Japan) are used with similar chromatography conditions as in example 1. All of these media are available with surface chemistries that allow attachment of EEL to the media. The coupling chemistries are based e.g. on Epoxy, Formyl, Carboxy, Tresyl or amino modifications of the surface, respectively. The chromatographic conditions are as described in Example 1.

Eluted fractions are also pooled and diafiltrated against PBS using a membrane with a MWCO of 30,000 Da (Millipore) to remove lactose and salts. Alternatively, diafltration can be performed in a Vivaspin 1 R Hydrosart with a MWCO of 30.000 Da (Sartorius).

### Example 4

The steps as described in example 3 are repeated, but with modified membrane adsorption chromatography.

Instead of applying lectin column chromatography, modified regenerated membrane filters are used as membrane adsorbers. Affinity membranes are prepared by immobilizing EEL to epoxy activated Sartobind Epoxy 75 membranes (Sartorius). In particular, the membrane unit is equilibrated with 20 ml of coupling buffer (1 M potassium phosphate buffer pH 8.0containing 0.1 mM CaCl₂ and MnCl₂ and 1.5 M lactose). Then 10 mg EEL is dissolved in 5 ml coupling buffer and circulated for 6 h at approximately 1 ml/min) over the membrane unit. Finally the membrane is washed with 20 ml PBS. Blocking of the unoccupied binding sites is not necessary. This membrane is than applied as for the affinity chromatography. Briefly, the sample is loaded to the EEL modified Sartobind Epoxy 75 membrane with a flow rate of 0.2 ml/min at room temperature. The target protein is eluted after non binding proteins are washed out in two steps with 0.5 M lactose in column loading buffer and 0.5 M lactose in addition to 1.850 M NaCi in column loading buffer with a flow rate of 0.5 ml/min at room temperature.

The eluted fractions are pooled and diafiltrated against phosphate buffered saline (PBS) using a membrane with a MWCO of 30,000 Da (Millipore) to remove lactose and salts. Alternatively, diafltration can be performed in a Vivaspin 1 R Hydrosart with a MWCO of 30.000 Da (Sartorius).

### Example 5

Purification of the viral membrane protein hemagglutinin from influenza by cellufine sulfate column chromatography.

Cultivation and inactivation is done as described in Example 1.

Then, polysorbate 80 (0,025 %, w/v) and cethyltrimethylammonium bromide (0.05 %, w/v) is added to the sediment and the mixture is incubated overnight at 4 °C while agitating to solubilize the viral membrane protein from the lipid membranes of the cellular and viral components.

Next, equimolar amounts of sodium cholate compared to cethyltrimethylammonium bromide are added to the mixture, thus, reducing the formation of micelles of the surfactants. The mixture is incubated for another 12 h at 4 °C while agitating.

Thereafter, the mixture is centrifugated for 10 min with 9000 g. Subsequently, the supernatant is diafiltrated against PBS using a membrane with a MWCO of 30,000 Da (Millipore) to concentrate the mixture and to remove salts and free surfactants.

Afterwards, the obtained solution is incubated with Amberlite XAD-4 to reduce the amount of surfactant. After performing the hydrophobic interaction chromatography with Amberlite XAD-4, the protein solution is centrifugated for 10 min at 9000 g.

As the next step, cellufine sulphate chromatography is performed. In particular, the sample is loaded to a 24 ml cellufine sulphate (Chisso Corporation) column with a flow rate of 0.5 ml/min in PBS at room temperature. The target protein is eluted after a column wash (PBS) with 1,2 M NaCl in PBS at room temperature.

The eluted fractions are pooled and diafiltrated against PBS using a membrane with a MWCO of 30,000 Da (Millipore) to remove salts. Alternatively, diafltration can be performed in a Vivaspin 1R Hydrosart with a MWCO of 30.000 Da (Sartorius).

### Example 6

Purification of the viral membrane protein hemagglutinin from influenza by sulfated membrane adsorption chromatography.

Samples are prepared as described in Example 5.

The sulphated reinforced cellulose membrane for the membrane adsorption chromatography is obtained as follows: To 20 ml pyridine, chlorosulfonic acid (1,12 ml) is added dropwise below 0 °C. After the addition, the mixture is heated to 65 °C while stirring and than cooled to 35 °C. 30 regenerated cellulose membranes (RC 55 membrane filter, 25 mm diameter, Whatman) are added to the cooled raction mixture and the reaction is continued under stirring at 35°C for 6 hours. After the reaction, the mixture is cooled to room temperature and neutralized with aqueous sodium hydroxide. Subsequently, the membrane is washed well with PBS.

The sample is loaded to 15 layers of the sulphated regenerated cellulose membranes in a stainless steel membrane holder (Whatman) with a flow rate of 0.5 ml/min in PBS at room temperature. The target protein is eluted after a column wash (PBS) with 1.2 M NaCl in PBS at room temperature.

The eluted fractions are pooled and diafiltrated against PBS using a membrane with a MWCO of 30,000 Da (Millipore) to remove salts. Alternatively, diafltration can be performed in a Vivaspin 1R Hydrosart with a MWCO of 30.000 Da (Sartorius).

### Example 7

Combination of the described process from example 1 and 5

Cultivation and inactivation is done as described in Example 1.

Then, polysorbate 80 (0,025 %, w/v) and cethyltrimethylammonium bromide (0.05 %, w/v) was added to the sediment and the mixture is incubated overnight at 4 °C while agitating to solubilize the viral membrane protein from the lipid membranes of the cellular and viral components.

Next, equimolar amounts of sodium cholate in relation to cethyltrimethylammonium bromide are added to the mixture, thus, reducing formation of micelles of the surfactants. The mixture is incubated for another 12 h at 4°C while agitating.

Thereafter, the mixture is centrifugated for 10 min with 9000 g. Subsequently, the supernatant is diafiltrated against PBS using a membrane with a MWCO of 30,000 Da (Millipore) to concentrate the mixture and to remove salts and free surfactants.

Afterwards, the obtained solution is incubated with Amberlite XAD-4 to reduce the amount of surfactant. After performing the hydrophobic interaction chromatography with Amberlite XAD-4, the protein solution is centrifugated for 10 min at 9000 g. As the next step, lectin-affinity chromatography is performed. In particular, the sample is loaded to a 3 ml EEL-polymer (Galab Technology GmbH) column with a flow rate of 0.2 ml/min at 4°C or at room temperature. The target protein is eluted after a column wash in two steps with 0.5 M lactose in column loading buffer and 0.5 M lactose in addition to 1.850 M NaCl in column loading buffer with a flow rate of 0.5 ml/min at 4°C or at room temperature.

The eluted fractions are pooled and diafiltrated against phosphate buffered saline (PBS) using a membrane with a MWCO of 30,000 Da (Millipore) to remove lactose and salts. Alternatively, diafltration can be performed in a Vivaspin 1 R Hydrosart with a MWCO of 30.000 Da (Sartorius).

In the following chromatography step the pooled and diafiltrated fractions from the EEL-polymer chromatography are loaded to a 24 ml cellufine sulphate (Chisso Corporation) column with a flow rate of 0.5 ml/min in PBS at room temperature. The target protein is eluted after a column wash (PBS) with 1.2 M NaCl in PBS at room temperature.

The eluted fractions are pooled and diafiltrated against PBS using a membrane with a MWCO of 30,000 Da (Millipore) to remove salts. Alternatively, diafltration can be performed in a Vivaspin 1R Hydrosart with a MWC4 of 30.000 Da (Sartorius).

### Example 8

The steps described in example 7 are conducted except that the order of the EEL-polymer chromatography and the cellufine sulphate chromatography is changed.

Comparing the yield of hemagglutinin from culture broth using the method according to the present invention with conventional methods wherein the supernatant of the culture broth is used only the presented examples allow an increase of hemagglutinin of the factor 2 to 5. The combination of two affinity chromatography methods as in examples 7 and 8 improves the purity of the target molecule. The column chromatography methods can also be freely exchanged by the membrane adsorption chromatography, allowing introducing to the chromatography process the advantages of membranes as e.g. reduced process time, reduced recovery volume, higher possible flowrates, ease of scale up and validation compared to classic column chromatography.

### Example 9

### Purification of whole inactivated virus

MDCK cells from ECACC, UK, Nr. 841211903 are initially grown in roller bottles (Greiner, Germany, 850 cm²) containing 250 ml GMEM basal medium (Invitrogen/Gibco, #22100-093). After the cells reach confluence they are washed with PBS, trypsinized and applied as an inocculum ((2-3) x10⁵ cells/ml) for a 5 I bioreactor (B. Braun Biotech) containing the same media and 2.0 g/l Cytodex 1 solid microcarriers (Amersham Biosciences). The cells are cultivated under standard conditions for 4 days, washed with PBS and infected with Influenza A PuertoRico/8/34, H1N1 (NIBSC #99-716) at an MOl of 0.025. The infected cultures are maintained for 48 to 72 h in virus maintenance medium under standard conditions as described in Genzel et al., Vaccine, 2004, 22, 2202-2208. After cultivation, cells and microcarriers as well as cell agglomerates are separated from the medium by sedimentation. The cultivation broth is than clarified by a combination of two filters (pore size: 5 and 0.65 µm, GE Water & Process Technologies) and subjected to chemical inactivation with 3 mM β-propiolactone (Serva Electrophoresis) for 24 h at 37°C. The inactivated broth is again clarified by a 0.45 µm membrane filter (GE Water & Process Technologies) and approximately 20-fold concentrated by a cross flow ultrafiltration (750 kDa; GE-Healthcare).

The concentrate is subjected to a 24 ml cellufine sulphate (Chisso Corporation) column with a flow rate of 0.5 ml/min in at room temperature. The virus is eluted after a column wash (PBS) with 1.2 M NaCl in PBS at room temperature.

The eluted fractions are pooled and diafiltrated against PBS using a membrane with a MWCO of 30,000 Da (Millipore) to remove salts.

The final chromatography step is based on the EEL-polymer affinity chromatography. The diafiltrated virus from the cellufine sulphate chromatography is loaded to a 3 ml EEL-polymer (Galab Technology GmbH) with a flow rate of 0.2 ml/min at 4°C or at room temperature. The virus is eluted after a column wash with 0.5 M lactose in column loading buffer with a flow rate of 0.5 ml/min at 4°C or at room temperature.

The eluted fractions are pooled and diafiltrated against phosphate buffered saline (PBS) using a membrane with a MWCO of 30,000 Da (Millipore) to remove lactose and salts. Alternatively, diafltration can be performed in a Vivaspin 1 R Hydrosart with a MWCO of 30.000 Da (Sartorius).

### Example 10

### Purification of heparin binding molecules with a sulfated reinforced cellulose membrane filter

Heparin binding molecules from cell culture, blood, serum or other sources can be captured or concentrated by a sulfated reinforced cellulose membrane filter.

Human serum is diluted 1:20 and subjected to to 15 layers of the sulphated regenerated cellulose membranes (prepared as described in example 6) in a stainless steel membrane holder (Whatman) with a flow rate of 0.2 ml/min at room temperature. Nonbinding proteins are eluted from the membrane with a flow rate of 0.2 ml/min PBS at room temperature. The binding proteins are than eluted by a linear gradient from 0 to 2 M NaCL and monitored with an online absorbance measurement at 280 nm. Individual peaks are dialysed against PBS and analysed by peptide mass fingerprint LC-MS.

## Claims

1. Method for obtaining isolated viral proteins from cultures containing host cells, comprising the steps of:
a) providing host cells containing viral proteins, optionally, with culture media;
b) adding at least one surfactant to the host cells of a) and incubating the same for membrane solubilization; and
c) purification of the viral proteins.

2. The method according to claim 1 wherein the viral protein is a viral membrane protein.

3. The method according to claim 1 or 2 further comprising the step of ultrafiltration in between step b) and c).

4. The method according to any one of claims 1 to 3 further comprising the step of demicellation before purification of the viral proteins.

5. The method according to any one of the preceding claims further comprising the step of protease treatment.

6. The method according to claim 5 wherein the protease treatment is a treatment with trypsin and/or bromelain,

7. The method according to any one of claims 5 or 6 wherein said protease treatment is conducted before ultrafiltration.

8. The method according to any one of the preceding claims wherein the purification of the viral proteins comprises the step of hydrophobic interaction chromatography.

9. The method according to any one of claims 1 to 8 further comprising the step of affinity separation by chromatography using a modified cellulose having sulfate or sulphate ester groups.

10. The method according to claim 9 wherein the affinity separation is an affinity membrane chromatography using sulphated cellulose membranes.

11. The method according to claim 9 wherein the affinity separation is an affinity column chromatography using a modified cellulose having sulphate or sulphate ester groups.

12. The method according to any one of the preceding claims further comprising the step of purification of the viral proteins with lectin-affinity chromatography.

13. The method according to claim 12 wherein the lectin is *Erythrina cristagalli* lectin or *Euonymus europaeus* lectin.

14. The method according to any one of claims 9 to 13 further comprising the step of ultrafiltration before the step of lectin affinity chromatography and/or after the step of lectin-affinity chromatography.

15. The method according to any one of claims 8 to 14 further comprising the step of heparin-affinity chromatography.

16. The method according to any one of claims 8 to 15 further comprising the step of ion exchange chromatography after hydrophobic interaction chromatography.

17. The method according to any one of claims 8 to 16 further comprising the step of sulfodextran affinity chromatography after hydrophobic interaction chromatography.

18. The method according to any one of claims 8 to 17 further comprising an affinity chromatography using specific monoclonal antibodies directed to the viral proteins to be purified.

19. The method according to any one of the preceding claims wherein the viral proteins are derived from single stranded RNA viruses, in particular, influenza virus, human immunodeficiency virus, herpes simplex virus.

20. The method according to any one of the preceding claims wherein the viral membrane proteins are viral proteins from influenza, virus, in particular, human influenza virus.

21. The method according to any one of the preceding claims wherein the host cells are virus infected host cells.

22. The method according to any one of claims 1 to 20 wherein the host cells are genetically engineered host cells expressing the viral protein of interest recombinantly.

23. The method according to any one of the preceding claims wherein the viral protein is hemagglutinin of influenza virus.

24. The method according to any one of the preceding claims wherein the surfactant at least one for membrane solubilization is a non-ionic, ionic or zwitter-ionic detergent.

25. The method according to any one of the preceding claims wherein a combination of at least two surfactants is added for membrane solubilization.

26. The method according to claim 24 wherein at least one surfactant is a non-ionic surfactant and at least one detergent is an ionic detergent.

27. The method according to any one of the preceding claims wherein the surfactant is a combination of cetyltrimethylammonium bromide as cationic surfactant and polysorbate as non-ionic surfactant.

28. Vaccine containing influenza surface antigen from influenza viruses wherein said influenza surface antigen is obtained by any one of the methods claims 1 to 27.

29. A method for the preparation of sulphated cellulose membranes comprising the steps of adding chlorosulfonic acid to pyridine at a temperature below 0°C; reacting said mixture at a temperature above 60°C; cooling the mixture at a temperature below 40°C; adding cellulose membrane, in particular regenerated cellulose membrane, to said mixture and reacting the same at a temperature below 40°C, thus, obtaining a sulphated cellulose membrane.
